# EUROPÄISCHE PATENTANMELDUNG

(11) **EP 2 722 014 A1**
(43) Veröffentlichungstag der Anmeldung: **23.04.2014**
(21) Anmeldenummer: 13188636.8
(22) Anmeldetag: 15.10.2013
(51) Int. Cl.: A61B 17/70

(54) **Osteosynthesevorrichtung**

(30) Priorität: 18.10.2012 DE 102012219050; 23.10.2012 DE 102012219350
(71) Anmelder: Silony Medical International AG, 8500 Frauenfeld (CH)
(72) Erfinder: Halm, Henry Prof. Dr., 23730 Neustadt (DE)
(74) Vertreter: DREISS Patentanwälte PartG mbB

(57) **Zusammenfassung**

Osteosynthesevorrichtung (1) mit einer Knochenschraube (12) mit einem eine Nut (22) aufweisenden Gabelkopf (18) mit zwei Schenkeln (20), z.B. für einen in die Nut des Gabelkopfes zwischen die beiden Schenkel einzulegenden Stützstab (14), wobei die Innenseiten der Schenkel des Gabelkopfes mit einem Innengewinde (26) versehen sind, das einen viereckigen oder trapezförmigen Querschnitt und eine Flanke aufweist, die, ausgehend von der Achse der Knochenschraube in radialer Richtung und in Richtung der freien Enden der Schenkel gesehen, derart geneigt ist, dass sich der Gewindequerschnitt erweitert.

## Beschreibung

Die Erfindung betrifft eine Osteosynthesevorrichtung mit einer Knochenschraube mit einem eine Nut aufweisenden Gabelkopf mit zwei Schenkeln, z.B. für einen in die Nut des Gabelkopfes zwischen die beiden Schenkel einzulegenden Stützstab, wobei die Innenseiten der Schenkel des Gabelkopfes mit einem Innengewinde versehen sind.

Aus der DE 43 16 542 C1 ist eine Knochenschraube bekannt geworden, mit der ein längsgenuteter Stab verdrehsicher fixiert werden kann. Hierfür weist die Knochenschraube einen Gabelkopf auf, in deren Nut der längsgenutete Stab eingelegt werden kann. Der Nutgrund ist mit Längsnuten versehen, welche eine formschlüssige Verbindung mit dem Stab herstellen. Zur Fixierung des Stabes wird auf den Gabelkopf eine Hutmutter aufgeschraubt und mit dieser der Stab im Nutgrund festgeklemmt. Durch die verdrehsichere Festlegung des Stabes an der Knochenschraube können hohe Korrekturkräfte bzw. Haltekräfte übertragen werden.

Es hat sich nun gezeigt, dass die Hutmutter zwar den Vorteil aufweist, dass beim Fixieren des längsgenuteten Stabes im Gabelkopf die beiden Schenkel des Gabelkopfes nicht nach außen aufgebogen werden können, da sie von der Hutmutter umgriffen werden. Hierfür benötigt die Knochenschraube jedoch entsprechend viel Platz.

Aus der DE 298 10 798 U1 ist eine Osteosynthevorrichtung bekannt, bei der der Stützstab nicht mittels einer Hutmutter sondern mittels einer Madenschraube im Gabelkopf festgehalten wird. Die Madenschraube besitzt ein Außengewinde und wird in ein Innengewinde des Gabelkopfes eingeschraubt und drückt mit ihrem eingeschraubten Ende auf den Stützstab und hält diesen am Nutgrund fest. Um zu vermeiden, dass beim Einschrauben und Festziehen der Madenschraube im Gabelkopf die beiden Schenkel des Gabelkopfes aufgebogen werden, weist das Innengewinde dieser Schenkel die Form eines Tannenbaumes auf, wobei die dem Nutgrund zugewandte Flanke des Gewindes von der Achse aus in radialer Richtung gesehen ansteigt. Die abzustützenden Kräfte, die von der Madenschraube auf den Gabelkopf übertragen werden, greifen an dieser in Richtung des Nutgrundes weisenden Flanke an. Da diese Flanke jedoch nach außen ansteigt wird der Gabelkopf nicht nach außen aufgebogen, sondern die beiden Schenkel werden von der Madenschraube eher nach innen gezogen.

Eine Osteosynthesevorrichtung mit den Merkmalen des Oberbegriffs des Anspruchs 1 ist bekannt aus US 2005/0216000 A1 und aus US 2010/0087874 A1.

Der Erfindung liegt die Aufgabe zugrunde, eine Osteosynthesevorrichtung der gattungsgemäßen Art derart weiterzubilden, dass mit der Madenschraube einerseits hohe Haltekräfte übertragen werden können, und andererseits aber keine gegenläufigen Spannungen in den Schenkeln des Gabelkopfs erzeugt werden.

Diese Aufgabe wird erfindungsgemäß mit einer Osteosynthesevorrichtung mit den Merkmalen des Anspruchs 1 gelöst.

Es wird der wesentliche Vorteil beibehalten, dass ohne die Verwendung einer Hutmutter oder eines anderen, die beiden Schenkel des Gabelkopfes umgreifenden Elements, die Schenkel selbst beim Einschrauben und Festziehen der Madenschraube nicht nach außen aufgebogen werden. Es werden Zugkräfte auf die Schenkel nur nach innen und nach oben, nicht aber nach unten erzeugt. Auf diese Weise können relativ hohe Kräfte von der Madenschraube auf den Stützstab übertragen werden. Zudem kann das Innengewinde relativ preiswert hergestellt werden. Eine ebene und zur Achse orthogonale untere Flanke erleichtert auch das Ansetzen und anschließende Einschrauben der Madenschraube, da der äußere Gewindegang der Madenschraube eben auf den auslaufenden Gewindegang des Gabelkopfes aufgesetzt werden kann.

Die vom Nutgrund abgewandte Flanke kann in radialer Richtung linear oder gebogen ansteigen. Die Biegung kann dabei progressiv oder degressiv verlaufen.

Ein weiteres erfindungsgemäßes Merkmal ist, dass die beiden Flanken über einen Gewindeabschnitt miteinander verbunden sind, der gerade oder gebogen verläuft. Dabei erstreckt sich der gerade Gewindeabschnitt bei einem Ausführungsbeispiel bevorzugt parallel zur Achse.

Bei einem anderen Ausführungsbeispiel der Erfindung nimmt der Abstand des Gewindeabschnitts ausgehend von der Achse der Knochenschraube in radialer Richtung und in Richtung der freien Enden der Schenkel gesehen, ab oder zu. Auf diese Weise wird der Abstand zweier Gewindegänge an deren Grund vergrößert, wodurch die Bruchgefahr oder die Gefahr eines Gewinderisses deutlich vermindert wird.

Eine Weiterbildung der Erfindung sieht vor, dass die in Richtung der Achse sich erstreckende Abmessung des Gewindeabschnitts maximal 75%, insbesondere 50% und bevorzugt 40% der Gewindesteigung entspricht. Hierdurch wird das Material in den Schenkeln des Gabelkopfes optimal genutzt.

Eine Fortbildung der Osteosynthesevorrichtung sieht vor, dass eine zwischen die Schenkel einzuschraubende Madenschraube vorgesehen ist, die ein in das Innengewinde eingreifendes Außengewinde aufweist, das einen rautenförmigen Querschnitt und eine vom Nutgrund abgewandte Flanke aufweist, die, ausgehend von der Achse der Madenschraube in radialer Richtung und in Richtung der freien Enden der Schenkel gesehen, geneigt ist. Mit dieser Madenschraube werden die beiden Schenkel des Gabelkopfes nicht aufgespreizt sondern aufeinander zu gezogen. Die beiden Flanken werden dabei bis zum Beginn der plastischen Verformung aufeinander gepresst, insbesondere dann, wenn die Flanken der Madenschraube und die Flanken des Gabelkopfes flächig aneinander anliegen.

Das Gewinde der Madenschraube ist komplementär zum Gewinde des Gabelkopfes.

Optimale Ergebnisse werden erzielt, wenn die geneigte Flanke mit einem Winkel vom 2° bis 15° geneigt ist. Kleine Neigungswinkel ergeben eine hohe Flächenpressung, die eine Selbsthemmung der Madenschraube bewirken.

Bevorzugt ist die Oberfläche wenigstens einer der Flanken glatt und/oder mit einem Gleitmittel beschichtet, so dass die Madenschraube leichtgängig eingeschraubt werden kann. Ferner ist bei einer bevorzugten Variante der Erfindung der Nutgrund im Übergang zur Flanke ausgerundet, wodurch die Rissbildung stark verringert wird.

Weitere Vorteile, Merkmale und Einzelheiten der Erfindung ergeben sich aus den Unteransprüchen sowie der nachfolgenden Beschreibung, in der unter Bezugnahme auf die Zeichnung besonders bevorzugte Ausführungsbeispiele im Einzelnen beschrieben sind. Dabei können die in der Zeichnung dargestellten und in den Ansprüchen sowie in der Beschreibung erwähnten Merkmale jeweils einzeln für sich oder in beliebiger Kombination erfindungswesentlich sein.

In der Zeichnung zeigen:
- Figur 1: einen Längsschnitt durch den Kopfbereich einer Knochenschraube mit eingelegtem Stützstab und eingeschraubter Madenschraube gemäß einem ersten Ausführungsbeispiel der Erfindung;
- Figur 2: eine vergrößerte teilweise Darstellung eines Innengewindes des Kopfbereichs einer Knochenschraube gemäß einem zweiten Ausführungsbeispiel der Erfindung.

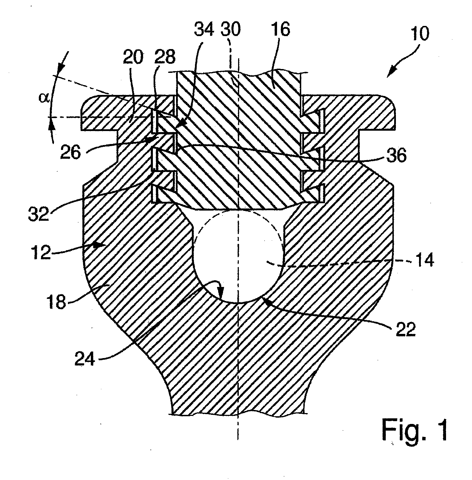

Die Figur 1 zeigt ein erstes Ausführungsbeispiel der erfindungsgemäßen, insgesamt mit 10 bezeichneten Osteosynthesevorrichtung mit einer Knochenschraube 12, einem lediglich andeutungsweise dargestellten Korrektur- oder Stützstab 14 sowie einer Madenschraube 16. Von der Knochenschraube 12 ist lediglich der Gabelkopf 18 dargestellt, welcher nach unten in den Gewindeteil übergeht, welcher das in den Knochen einzuschraubende Gewinde aufweist. Der Gewindeteil kann einstückig vom Gabelkopf abragen oder als separates Bauteil ausgeführt sein.

Der Gabelkopf 18 weist zwei Schenkel 20 auf. Die beiden Schenkel 20 bilden eine Nut 22, welche einen Nutgrund 24 aufweist. Dieser Nutgrund 24 ist üblicherweise mit einer Oberflächenprofilierung versehen und dient auch gegebenenfalls zur Aufnahme eines Druckstücks für den separaten Gewindeteil und zur Aufnahme für den Korrektur- und Stützstab 14, der von der Madenschraube 16 festgehalten wird. Diese Madenschraube 16 ist in den Gabelkopf 18 eingeschraubt, der hierfür ein insgesamt mit 26 bezeichnetes Innengewinde aufweist. Dieses Innengewinde 26 ist im Querschnitt trapezförmig ausgestaltet, wobei die vom Nutgrund 24 abgewandte obere Flanke 28 von der Achse 30 der Knochenschraube 12 in radialer Richtung gesehen ansteigt. Der Neigungswinkel α der oberen Flanke 28 ist also größer Null und beträgt zwischen 5° und 10°, insbesondere 8°. Die untere Flanke 32 des Innengewindes 26 verläuft orthogonal zur Achse 30.

Die Madenschraube 16 weist ein insgesamt mit 32 bezeichnetes Außengewinde auf, welches zum Innengewinde 26 des Gabelkopfes 18 korrespondiert, insbesondere komplementär zu diesem ist.

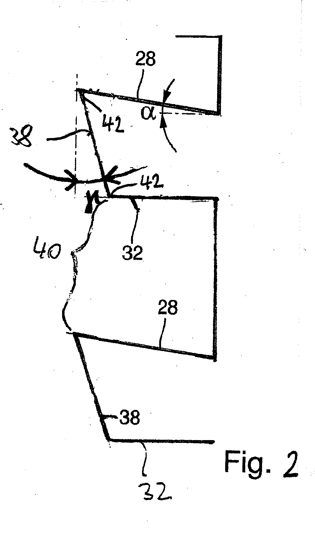

Die Figur 2 zeigt einen vergrößerten Ausschnitteines Innengewindes gemäß einer weiteren Ausführungsform der Erfindung. Dabei ist deutlich der zur Achse 30 um den Winkel γ geneigte Gewindeabschnitt 38 erkennbar. Außerdem ist das Innengewinde 26 im Übergang 42 vom Gewindeabschnitt 38 zu den Flanken 28 und 32 gerundet. Die untere Flanke 32 erstreckt sich orthogonal zur Achse 30. Der Abstand des zur Achse 30 um den Winkel γ geneigten Gewindeabschnitts 38 nimmt in der Figur 2, ausgehend von der Achse 30 der Knochenschraube 12 in radialer Richtung und in Richtung der freien Enden der Schenkel 20 gesehen, zu.

## Patentansprüche

1. Osteosynthesevorrichtung (10) mit einer Knochenschraube (12) mit einer Schraubenachse (30) und einer hierzu radialen Richtung, wobei die Knochenschraube (12) mit einem eine Nut (22) aufweisenden Gabelkopf (18) mit zwei Schenkeln (20) ausgebildet ist, z.B. für einen in die Nut (22) des Gabelkopfes (18) zwischen die beiden Schenkel (20) einzulegenden Stützstab (14), wobei die Innenseiten der Schenkel (20) des Gabelkopfes (18) mit einem Innengewinde (26) versehen sind, wobei das Innengewinde (26) in einer die Achse (30) einschließenden Ebene betrachtet einen viereckförmigen sich in radialer Richtung erweiternden Querschnitt mit einer dem Nutgrund (24) zugewandten linearen Flanke (32) und einer vom Nutgrund (24) abgewandten Flanke (28) aufweist, **dadurch gekennzeichnet, dass** die dem Nutgrund (24) zugewandte Flanke (32) in der die Achse (30) einschließenden Ebene betrachtet in radialer Richtung, also orthogonal zu der Achse (30) verläuft und dass die vom Nutgrund (24) abgewandte Flanke (28) zur radialen Richtung in Richtung der freien Enden der Schenkel (20) geneigt ist.

2. Osteosynthesevorrichtung nach Anspruch 1, **dadurch gekennzeichnet, dass** die vom Nutgrund (24) abgewandte Flanke (28) in radialer Richtung linear geneigt oder gebogen ist, insbesondere degressiv oder progressiv gebogen ist.

3. Osteosynthesevorrichtung nach Anspruch 1 oder 2,
**dadurch gekennzeichnet, dass** die beiden Flanken (28, 32) über einen Gewindeabschnitt (38) miteinander verbunden sind, der gerade oder gebogen verläuft.

4. Osteosynthesevorrichtung nach Anspruch 3, **dadurch gekennzeichnet, dass** der gerade Gewindeabschnitt (38) sich parallel zur Achse (30) erstreckt, oder dass der Abstand des Gewindeabschnitts (38) zur Achse (30) der Knochenschraube (12), in Richtung der freien Enden der Schenkel (20) gesehen, ab- oder zunimmt.

5. Osteosynthesevorrichtung nach einem der Ansprüche 3 oder 4, **dadurch gekennzeichnet, dass** die in Richtung der Achse (30) sich erstreckende Abmessung des Gewindeabschnitts (38) maximal 75%, insbesondere 50% und bevorzugt 40% der Gewindesteigung entspricht.

6. Osteosynthesevorrichtung nach einem der vorhergehenden Ansprüche, **dadurch gekennzeichnet, dass** eine zwischen die Schenkel (20) einzuschraubende Madenschraube (16) vorgesehen ist, die ein in das Innengewinde (26) eingreifendes Außengewinde (34) aufweist, das einen rautenförmigen Querschnitt und eine vom Nutgrund (24) abgewandte Flanke (36) aufweist, die ausgehend von einer Achse (30') der Madenschraube (16), die im eingeschraubten Zustand der Madenschraube (16) mit der Achse (30) der Knochenschraube (12) koinzidiert, in der die Achse (30) einschließenden Ebene betrachtet zur radialen Richtung in Richtung der freien Enden der Schenkel (20) geneigt ist.

7. Osteosynthesevorrichtung nach Anspruch 6, **dadurch gekennzeichnet, dass** die Flanken (36) der Madenschraube (16) und die Flanken (28, 32) des Gabelkopfes (18) flächig aneinander anliegen.

8. Osteosynthesevorrichtung nach einem der vorhergehenden Ansprüche, **dadurch gekennzeichnet, dass** die Flanke (32, 36) mit einem Winkel (α, β, γ) vom 2° bis 15°, insbesondere 3° bis 10°, bevorzugt 4° bis 5° geneigt ist.

9. Osteosynthesevorrichtung nach einem der vorhergehenden Ansprüche, **dadurch gekennzeichnet, dass** dass die beiden Flanken (28, 32) des Innengewindes (26) über einen Gewindeabschnitt (38) miteinander verbunden sind, dessen Abstand zur Achse (30) der Knochenschraube (12), in Richtung der freien Enden der Schenkel (20) gesehen, zunimmt.

10. Knochenschraube (12) einer Osteosynthesevorrichtung (10) nach einem der vorhergehenden Ansprüche.
